# EUROPÄISCHE PATENTSCHRIFT

(11) **EP 1 233 713 B1**
(45) Veröffentlichungstag und Bekanntmachung des Hinweises auf die Patenterteilung: **18.08.2004**
(21) Anmeldenummer: 00972856.9
(22) Anmeldetag: 24.10.2000
(51) Int. Cl.: A61B 17/80

(54) **IMPLANTAT FÜR OSTEOSYNTHESEN**
IMPLANT FOR OSTEOSYNTHESES
IMPLANT DESTINE A DES OSTEOSYNTHESES

(30) Priorität: 27.10.1999 DE 19951760
(43) Veröffentlichungstag der Anmeldung: 28.08.2002
(73) Patentinhaber: Sepitec Foundation, 9490 Vaduz (LI)
(72) Erfinder: DRANSFELD, Clemens, CH-5702 Niederlenz (CH); MAGERL, Fritz, CH-9011 St. Gallen (CH); TOGNINI, Roger, Roland, CH-9443 Widnau (CH); PETER, Thomas, Andreas, 4434 Hölstein (CH)
(74) Vertreter: Menges, Rolf, Dipl.-Ing.
(86) Internationale Anmeldenummer: PCT/EP2000/010465
(87) Internationale Veröffentlichungsnummer: WO 2001/030251

(56) Entgegenhaltungen:
- WO-A-89/04150
- WO-A-97/09000
- DE-U- 8 628 766
- FR-A- 742 618
- US-A- 5 147 361

## Beschreibung

Die Erfindung betrifft ein Implantat für Osteosynthesen, bestehend aus einer mit mehreren in Längsrichtung aufeinander folgenden Löchern versehenen Platte und durch die Löcher in der Platte hindurch einsetzbaren und in bestimmungsgemäßer Lage in einen Knochen eindrehbaren Schrauben, wobei zumindest ein Größteil der zur Aufnahme der Schrauben bestimmten Löcher bezogen auf eine gedachte Mittelebene der Platte alternierend nach außen versetzt sind, wobei die Mittelachsen der Löcher mit der gedachten Mittelebene der Platte einen spitzen Winkel einschließen und wobei die Löcher von der bestimmungsgemäß außen liegenden Oberfläche der Platte ausgehend sich verjüngen.

Bei einer bekannten Knochenplatte (DE 86 28 766 U) sind seitlich zur Mittellängsachse versetzte Löcher vorgesehen, in welche Schrauben einsetzbar sind. Wenn die Löcher jeweils um einen größeren Winkelbetrag von 10 bis 20° (bezogen auf den runden Knochenquerschnitt) aus der Mittelachse versetzt sind, ist die Platte zur Anpassung an die Knochenoberfläche um eine zur Längsrichtung parallele Achse gekrümmt oder in sonstiger Weise (insbesondere durch polygonalen Querschnitt) einer zylindrischen Form angenähert. Dadurch, dass die Querabmessung der Knochenplatte trotz der seitlich versetzten Anordnung der Löcher, örtlich betrachtet, nicht wesentlich verbreitert werden muss, lässt sich die Platte mittels üblicher Werkzeuge verformen und dem Knochverlauf individuell anpassen. Die Breite der Platte entspricht dabei durchgehend in etwa der Breite einer normalen schmalen Platte, wobei der Verlauf dieser Platte durch die Ausnehmungen einen zickzackförmigen Charakter erhält. Damit kann die Platte auch eine schraubenförmige Verwindung erhalten, so dass je nach Art der Fraktur die optimalen Befestigungspunkte in diesem Fall erreicht werden können. Weil in dem Stand der Technik, von dem bei dieser bekannten Knochenplatte ausgegangen wird, die Platten durchgehend relativ breit und somit nur schwer durch Verformung an die jeweiligen Fraktursituationen anpassbar sind, wird bezweckt, die bekannte Knochenplatte so auszubilden, dass sie durch eine individuelle Formgebung an eine größere Zahl von Frakturtypen anpassbar ist.

Weiter ist eine Knochenplatte bekannt geworden (EP 0 206 767 A), bei der die Löcher seitlich zur Mittellängsachse versetzt angeordnet sind, wobei die Löcher Ansenkungen im wesentlichen in Form eines Abschnittes einer Kugel aufweisen und die Unterseite des Kopfes der Schrauben eine korrespondierende Querschnittsform haben. Dadurch kann der Schraubenkopf immer satt in der Ansenkung der Knochenplatte anliegen. Eine winkelstabile Lage zwischen Knochenplatte und Schraube ist hier aber nicht zu erzielen.

Bei einer anderen bekannten Knochenplatte (WO 97/09000 A) sind wohl konisch sich verjüngende Löcher vorhanden und korrespondierend ausgebildete Köpfe der einzusetzenden Schrauben, jedoch kann bei parallelachsig verlaufenden Schrauben keine dauerhaft winkelstabile Verbindung erzielt werden. Ein Großteil der Kräfte muss hier also von der Platte durch direktes Anpressen auf der Knochenoberfläche übertragen werden.

Bei dem bisher eingesetzten mechanischen Prinzip der konventionellen Plattenosteosynthese liegt die Platte flächig oder mit vorstehenden Leisten oder Nocken direkt an den Knochen an. Für die Übertragung der Kräfte von einem Knochenfragment auf das andere ist daher in der Regel der Anpressdruck der Platte an den Knochen und die dadurch hervorgerufene Reibung der Platte an dem Knochen entscheidend. Es ergibt sich dadurch eine direkte Übertragung der Kräfte vom Knochen auf die Platte und von dieser wieder auf den Knochen. Sobald eine eingesetzte Schraube sich lockert, also geringfügig zurückdreht, versagt dieses Stabilisierungsprinzip. Aber auch in biologischer Hinsicht ergeben sich hier Probleme. Infolge von Durchblutungsstörungen ergibt sich eine Nekrosezone unter der Platte. Die Gefäße der Knochenhaut - des Periosts - werden abgeklemmt.

Die vorliegende Erfindung hat sich zur Aufgabe gestellt, ein Implantat der eingangs genannten Art zu schaffen, mit welchem die Kraftübertragung über eine Platte sowohl aus mechanischer als auch biologischer Sicht wesentlich verbessert werden kann.

Erfindungsgemäß gelingt dies dadurch, dass die Löcher der Platte sich konisch verjüngen. dass die Schrauben einen zu den von den Löchern gebildeten Abschnitten korrespondierenden, sich konisch zu dein mit einem Gewinde versehenen Schaft hin verjüngenden Kopf aufweisen, der kraft- und/oder formschlüssig in den Löchern fixierbar ist, dass die Platte in deren Längsrichtung gesehen mehrfach verwunden ausgeführt ist, wobei im Bereich der einzelnen Löcher die Hauptausrichtung quer zur Längserstreckung der Platte jeweils rechtwinklig zur Mittelachse des entsprechenden Loches verläuft, und dass die Platte von der Seite her gesehen leicht gebogen ausgeführt ist, wobei eine durch die Enden der Platte gelegte Sehne Abstand von einem Mittelabschnitt der Platte hat.

Durch diese erfindungsgemäßen Maßnahmen wird erreicht, dass die Platte nicht mehr mit einem Pressdruck an den Knochen anliegt. Es ergibt sich sogar zwischen den Knochen und der Platte ein Spalt. Die Übertragung der Kräfte von einem Fragment auf das andere ergibt sich nun wie folgt: Knochen - Schraubenschaft - Kopf der Schraube(n) - platte - Kopf der Schraube(n) - Schraubenschaft - Knochen. Voraussetzung dazu war die durch die Erfindung geschaffene Möglichkeit, dass nämlich eine winkelstabile Klemmverbindung zwischen dem Kopf der Schrauben und der Platte sich ergibt. Durch die winkelstabile Klemmverbindung des Kopfes der Schraube in dem entsprechenden Loch in der Platte werden eine optimale Winketstabitität und eine Rückdrehsicherung geschaffen. Eine solche Rückdrehsicherung ist insbesondere deshalb vorteilhaft, weil ein Rückdrehen allein schon bei repetitiven Wechsellasten auftreten könnte.

Neben einer sicheren Stabilisierung ist es dadurch gegebenenfalls auch möglich, mit weniger Schrauben auszukommen. Unter der Platte ergeben sich so keine oder wesentlich weniger Durchblutungsstörungen, da die Gefäße der Knochenhaut nicht abgeklemmt werden.

Die Bohrungen in den Knochen für den Einsatz von Schrauben sind nicht in einer Reihe ausgerichtet. Die Fragmentspaltung ist dadurch im wesentlichen verhindert und außerdem ergeben sich dadurch auch wesentlich weniger Durchblutungsstörungen. Besonders vorteilhaft wirkt sich dabei die Konvergenz der eingesetzten Schrauben aus. Die aufeinanderfolgend an einem Knochen eingesetzten Schrauben kreuzen sich somit annähernd im Zentrum des Markraumes im Knochen. Durch diese Maßnahme wird die Torsionsstabilität der Plattenösteosynthese wesentlich erhöht. In einer Linie relativ nahe zueinander implantierte Schrauben können bei Einwirkung starker Torsionskräfte eine Spaltung des Knochens bewirken. In einer Linie relativ nahe zueinander implantierte Schrauben können ferner durch Unterbrechung der in den längs gerichteten Haver'schen Kanälen verlaufenden Blutgefäße eher eine sich ungünstig auswirkende Durchblutungsstörung verursachen als zueinander versetzte Löcher und somit als einander aufeinanderfolgend kreuzende Schrauben.

Durch die erfindungsgemäßen Maßnahmen ist ein Schritt in eine elastische Platte erreicht worden, wobei durch die Bewegungsmöglichkeiten eine natürliche Knochenheilung mit Callusbildung erwartet wird, wie dies früher eben bei der Anwendung eines Gipsverbandes der Fall war.

Dadurch, dass erfindungsgemäß die Platte in deren Längsrichtung gesehen mehrfach verwunden ausgeführt ist, wobei im Bereich der einzelnen Löcher die Hauptausrichtung quer zur Längserstreckung der Platte jeweils rechtwinklig zur Mittelachse des entsprechenden Loches verläuft, liegt die Platte im jeweiligen Befestigungsbereich annähernd parallel zur Oberfläche des Knochens. Es ist somit im wesentlichen immer ein gleichbleibender Spalt zwischen Knochen und Platte gegeben.

Weiter wird vorgeschlagen, dass die Seitenbegrenzungen der Platte im wesentlichen den versetzten Löchern und den Außenkonturen der Löcher folgen, so dass die Platte über deren Länge in Draufsicht gesehen einen im wesentlichen wellenförmigen Verlauf aufweist. Dadurch ist über die Länge der Platte zumindest annähernd eine gleichbleibende Stabilität trotz einer materialsparenden Fertigung möglich geworden. Es ist somit auch die Gewähr gegeben, dass die einzusetzende Platte nicht überdimensioniert ist.

Um gerade am Übergangsbereich zwischen zwei miteinander zu verbindenden Knochenfragmenten eine exakte Ausrichtung zu ermöglichen, wird vorgeschlagen, dass die dem Mittelabschnitt der Platte bezogen auf deren Länge nächstliegenden beiden Löcher derselben Seitenbegrenzung der Platte zugewandt sind.

In diesem Zusammenhang ist es auch vorteilhaft, wenn die gedachte Mittelebene der Platte auch die Mittelebene des Mittelabschnittes der Platte ist. Dies erleichtert die Zentrierung der Platte am Knochen.

Eine optimale konstruktive Gestaltung ergibt sich dadurch, dass die Größe der Querschnittsfläche der Platte zumindest annähernd über deren ganze Länge konstant ist. Es ergeben sich dadurch keine durch die Löcher in der Platte verursachten Schwachstellen.

Da gerade im Verbindungsbereich zwischen zwei Knochen, also in dem beispielsweise einen Knochenbruch überbrückenden Bereich besondere Kräfte von der Platte zu übertragen sind, ist es jedoch vorteilhaft, wenn die Querschnittsfläche der Platte im Bereich des Mittelabschnittes größer ausgeführt ist als in den anderen Abschnitten der Platte.

Um in jedes Knochenfragment die Kräfte über die Schrauben in gleichem Maße einbringen zu können, wird vorgeschlagen, dass der Abstand der Löcher in Längsrichtung der Platte gesehen von den Plattenenden ausgehend gleich ist, daß jedoch der Abstand zwischen den beiden an den Mittelabschnitt anschließenden Löchern größer ist. Durch den größeren Abstand der beiden fraktumahen Schrauben von der Frakturfläche werden Durchblutungsstörungen vermindert. Im Knochen verlaufen die Hauptblutgefäße in Längsrichtung. Wenn somit die fraktumahen Schraubenlöcher zu nahe der Frakturfläche sind, könnte es eine "schattenförmige" Durchblutungsstörung geben.

Weiter wird vorgeschlagen, dass an der Unterseite der Platte ein- oder beidseitig eines jeden Loches nahe der Seitenbegrenzungen auskragende Nocken ausgebildet sind. Diese Nocken bilden "Füßchen" an den Plattenrändern, die hauptsächlich bei der Plattenmontage vorteilhaft sein können. Diese Nocken verhindern ein vollflächiges Anliegen der Platte am Knochen, können gegebenenfalls die Torsionsstabilität erhöhen bzw. die Schraubenhälse am Übergang zwischen Schraubenschaft und Kopf entlasten. Durch die Nocken allein wird die Durchblutung des Knochens nicht wesentlich gestört. Durch die Verktemmung der Schrauben in der Platte selbst wird erreicht, dass die Platte schlussendlich einen gewissen Abstand von der Oberfläche des Knochens beibehält

Zur Verhinderung von Gewebeschäden an den Weichteiten, speziell an den Sehnen, die über der Platte liegen oder über die Platte ziehen, wird vorgeschlagen, dass die beiden Enden und Kanten und Übergänge der Platte flach und abgerundet ausgeführt sind.

Eine weitere spezielle Ausgestaltung sieht vor, dass die Mittelachsen der Löcher mit der gedachten Mittelebene der Platte einen spitzen Winkel von 15° einschließen. Durch das spitzwinklige Eindrehen der Schrauben und die sich kreuzenden Löcher samt Schrauben wird die Torsionsstabilität der Osteosynthese wesentlich verbessert. Die Schrauben werden in wesentlich geringerem Maße auf Biegung beansprucht.

Weiter wird erfindungsgemäß vorgeschlagen, dass die Platte aus faserverstärkten Thermoplasten hergestellt ist und die Anisotropie der elastischen Eigenschaften der Platte auf die Elastizität oder Steifigkeit des Knochens eingestellt ist. Das erfindungsgemäße Osteosyntheseplattensystem ist somit als elastische Fixation ausgelegt. Es ist somit eine Homoelastizität gegeben, weil die Platte nur eine gleichartige Steifigkeit und nicht wie diese für isoelastische Implantate gefordert wird, eine gleichwertige Steifigkeit hat Durch den Einsatz eines solchen Werkstoffes und durch die entsprechende Fertigung ergibt sich der wesentliche Vorteil einer elastischeren Osteosynthese. Dadurch ergibt sich weniger Stressshielding und weniger reaktive Osteoporose. Außerdem wird der Anreiz für eine Callusbildung gefördert. Gerade durch die Kombination einer Platte aus faserverstärkten Thermoplasten und einer Verklemmung zwischen Kopf der Schraube und der Lochwandung in der Platte wird eine elastische Platte für eine optimale natürliche Knochenheilung erzielt.

In diesem Zusammenhang ist es besonders vorteilhaft, dass die Anisotropie der elastischen Eigenschaften der Platte gemäß der Formel E-Modul(längs) : E-Modul(tagential) = 0.3 bis 0,7 eingestellt ist. Der E-Modul einer solchen homoelastischen Platte bewegt sich zwischen 30 und 70 GPa (bei Knochen sind dies bis 20 GPa). Schon bei einer Evaluationsplatte ergab sich ein Verhältnis gemäß der genannten Formel von ca. 0,3. Als System, also auf Knochenersatz montiert, war bei der Evaluationsplatte dieses Verhältnis noch kleiner. Die versetzten Schrauben bringen dieses Verhältnis auf einen guten Mittelwert von ca. 0,5. Die Feineinstellung dieser anisotropen elastischen Eigenschaften kann durch entsprechende Steuerung im Herstellungsverfahren, beispielsweise in einem Gegentaktfließpressverfahren, erfolgen.

Eine weitere vorteilhafte Maßnahme wird darin gesehen, dass der Kopf der Schraube mit einem Gewinde versehen ist. In bevorzugter Weise ist dabei der Kopf der Schraube mit einem Feingewinde versehen. Dadurch ist eine optimale Vorspannung der Schraube in der Platte möglich geworden. Heute eingesetzte Osteosyntheseplatten werden gegenüber dem Knochen vorgespannt. Die so in den Knochen induzierten Spannungen können zu Knochenresorption, Knochenabbau und somit zu Knochenschwächung führen, was wiederum nach Entfernen des Implantates das Risiko einer Refraktur erhöht. Dank der Vorspannung der Schrauben in der Platte selbst wird keine Druckbelastung von der Platte aus in den Knochen induziert, was einen höheren Heilungserfolg verspricht.

Durch den Einsatz eines Gewindes, vorzugsweise eines gegebenenfalls zweigängigen Feingewindes ergibt sich ein optimales Verklemmen des Kopfes der Schraube in dem entsprechenden Loch in der Platte. Durch den leicht konischen, mit einem Feingewinde versehenen Kopf ist einerseits das Einführen des Schraubenkopfes in das entsprechende Loch in der Platte erleichtert, was bei einer leicht exzentrischen Bohrung des Schraubenkanals im Knochen wichtig sein könnte, andererseits ergibt sich eine wirksame Verklemmung des Kopfes in dem entsprechenden Loch.

Gerade durch den Einsatz eines Gewindes, insbesondere eines Feingewindes, am Kopf der Schraube wird auch eine gute Sicherung gegen ein Rück- oder Ausstoßen des Kopfes aus dem Loch geschaffen. Solche axiale Kräfte entstehen bei einer Krafteinwirkung in Schraubenlängsachse vom Knochen gegen den Kopf der Schraube, z.B. bei einer Torsions- oder Biegebeanspruchung.

Eine vorteilhafte Maßnahme wird auch darin gesehen, dass der Kopf der Schraube im Bereich des Gewindes mit einer oder mit mehreren Längsrille(n) versehen ist. Dadurch wird die Möglichkeit der Aufnahme von Gewebedebris geschaffen.

Für eine zusätzliche Verdrehsicherung der Schraube ist vorgesehen, dass die Schraube an ihrem mit einem Gewinde versehenen Schaft im Querschnitt unrund, z.B. trilobular, ausgeführt ist. Nach dem Eindrehen der Schraube bewirkt das nachwachsende Knochengewebe sozusagen eine Eigensicherung, da die unrunde Schraube eingespannt wird. Es ist damit auch im Knochen selbst eine Art elastischer Verklemmung gegeben.

Damit das große Drehmoment beim Eindrehen der Schrauben, aber auch beim Herausdrehen derselben bestmöglich übertragen werden kann, wird vorgeschlagen, dass der Kopf der Schraube mit einem Innenwerkeugangriff mit vier von einer mittigen Öffnung radial nach außen geführten bogenförmigen Ausbuchtungen versehen ist.

Weitere erfindungsgemäße Merkmale und besondere Vorteile werden in der nachstehenden Beschreibung anhand der Zeichnungen noch näher erläutert. Es zeigen:
Fig. 1 eine Schrägansicht einer als Implantat für eine Osteosynthese einsetzbaren Platte;
Fig. 2 eine Ansicht einer als Implantat für eine Osteosynthese einsetzbaren Schraube;
Fig. 3 einen Schnitt durch ein Einsatzbeispiel des Implantates aus Platte und Schraube an einem Knochen;
Fig. 4 eine Draufsicht auf eine Platte;
Fig. 5 einen Schnitt nach der Linie V-V in Fig. 4;
Fig. 6 einen Horizontalschnitt nach der Linie VI-VI in Fig. 7;
Fig. 7 eine Seitenansicht der Platte;
die Fig. 8 bis 15 Schnitte nach den Linien VIII-VIII bis XV-XV in Fig. 7.

Das in den Zeichnungen dargestellte Implantat für Osteosynthesen besteht aus einer mit mehreren in Längsrichtung aufeinander folgenden Löchern 3 versehenen Platte 1 und meist mehreren durch die Löcher 3 in der Platte 1 hindurch einsetzbaren und in bestimmungsgemäßer Lage in einen Knochen 4 eindrehbaren Schrauben 2. Zumindest ein Großteil der zur Aufnahme der Schrauben 2 bestimmten Löcher 3 sind bezogen auf eine gedachte Mittelebene 5 der Platte 1 alternierend nach außen versetzt angeordnet Die Mittelachsen 6 der Löcher 3 schließen mit der gedachten Mittelebene 5 der Platte 1 einen spitzen Winkel W ein. Die Löcher 3 verjüngen sich von der bestimmungsgemäß außen liegenden Oberfläche 7 der Platte 1 ausgehend konisch. Die Schrauben 2 weisen einen zu den von den Löchern 3 gebifdeten Abschnitten im wesentlichen korrespondierenden, sich konisch zu dem mit einem Gewinde 8 versehenen Schaft 9 hin verjüngenden Kopf 10 auf. Die Schraube 2 ist über den Kopf 10 kraft- und/oder formschlüssig in den Löchern 3 fixierbar.

Die Seitenbegrenzungen 11, 12 der Platte 1 folgen im wesentlichen den versetzten Löchern 3 und auch. den Außenkonturen der Löcher 3. Die Platte 1 weist daher über deren Länge in Draufsicht gesehen einen im wesentlichen wellenförmigen Verlauf auf. Ferner ist die Platte 1 in deren Längsrichtung gesehen mehrfach verwunden ausgeführt, wobei im Bereich der einzelnen Löcher 3 die Hauptausrichtung quer zur Längserstreckung der Platte 1 jeweils zumindest annähernd rechtwinklig zur Mittelachse 6 des entsprechenden Loches 3 verläuft.

Die einem Mittelabschnitt 13 der Platte bezogen auf deren Länge nächstliegenden beiden Löcher 3 sind derselben Seitenbegrenzung 11 (oder 12) der Platte 1 zugewandt. Der Begriff "Mittelabschnitt" bezeichnet zwar einen zentralen Teil der Platte, der die Mitte der Platte enthält, jedoch muss dieser Mittelabschnitt 13 nicht immer genau in der Mitte bezogen auf die Länge der Platte 1 angeordnet sein. Bei Platten mit einer geraden Anzahl von Löchern 3 ist es wohl immer die Mitte (außer bei extrem langen Platten). Bei einer ungeraden Anzahl von Löchern 3 liegt der Mittelabschnitt 13 zwischen den Löchern x/2+0,5 und x/2-0,5. Vorteilhaft ist dabei die Konstruktion so, dass die gedachte Mittelebene 5 der Platte 1 auch die Mittelebene 5 des Mittelabschnittes 13 der Platte 1 ist.

Von der Seite her gesehen ist die Platte 1 leicht gebogen ausgeführt, wobei beispielsweise der Abstand einer durch die Plattenenden 14 gelegten Sehne vom Mittelabschnitt 13 etwa 2 mm betragen kann. Diese Längsbiegung kann auch dem Aufbiegen einer Fraktur entgegenwirken, wenn ein Biegemoment senkrecht auf die Unterseite der Platte einwirkt. Es ist durch diese Längsbiegung eine bessere Anpassung an die Geometrie des Unterarmknochens möglich geworden.

Die Größe der Querschnittsfläche der Platte 1 ist zumindest annähernd über deren ganze Länge konstant. Die Querschnittsfläche der Platte 1 im Bereich des Mittelabschnittes 13 kann jedoch größer ausgeführt sein als in den anderen Abschnitten der Platte 1. Gerade In diesem den Frakturbereich übergreifenden Mittelabschnitt ist somit eine zusätzliche Optimlerung der Torsionssteifigkeit möglich.

Der Abstand der Löcher 3 in Längsrichtung der Platte 1 gesehen von den Plattenenden 14 ausgehend ist gleichbleibend. Es kann jedoch der Abstand zwischen den beiden an den Mittelabschnitt 13 anschließenden Löchern 3 größer sein. Je nach Einsatzbereich oder besonderer Umstände ist es auch denkbar, die Lochabstände variabel zu gestalten.

An der Unterseite 16 der Platte 1 können ein- oder beidseitig eines jeden Loches 3 nahe der Seitenbegrenzungen 11, 12 auskragende Nocken 17 ausgebildet sein. Diese Nocken 17 können bei der Montage der Platte vorteilhaft sein, um dadurch einen entsprechenden Spalt zwischen der Knochenoberfläche und der Platte 1 herzustellen. Im Endzustand wird jedoch die Platte 1 nicht gegen die Oberfläche des Knochens 4 gepresst, so dass die Nocken ohne Druck aufliegen und praktisch nur den Abstand bei der Montage selbst sichern. Durch das Eindrehen der Schrauben selbst erfolgt in keiner Weise ein Anpressen der Platte an die Oberfläche des Knochens.

Die beiden Enden 14 der Platte 1, aber auch alle Kanten und Übergänge, sind flach und abgerundet ausgeführt. Die in der Zeichnung noch annähernd zylindrisch dargestellten Ansätze 15 sind lediglich aus Herstellungsgründen noch angebrachte Abschnitte, welche jedoch in der Regel schon vor dem endgültigen Einsatz abgetrennt werden.

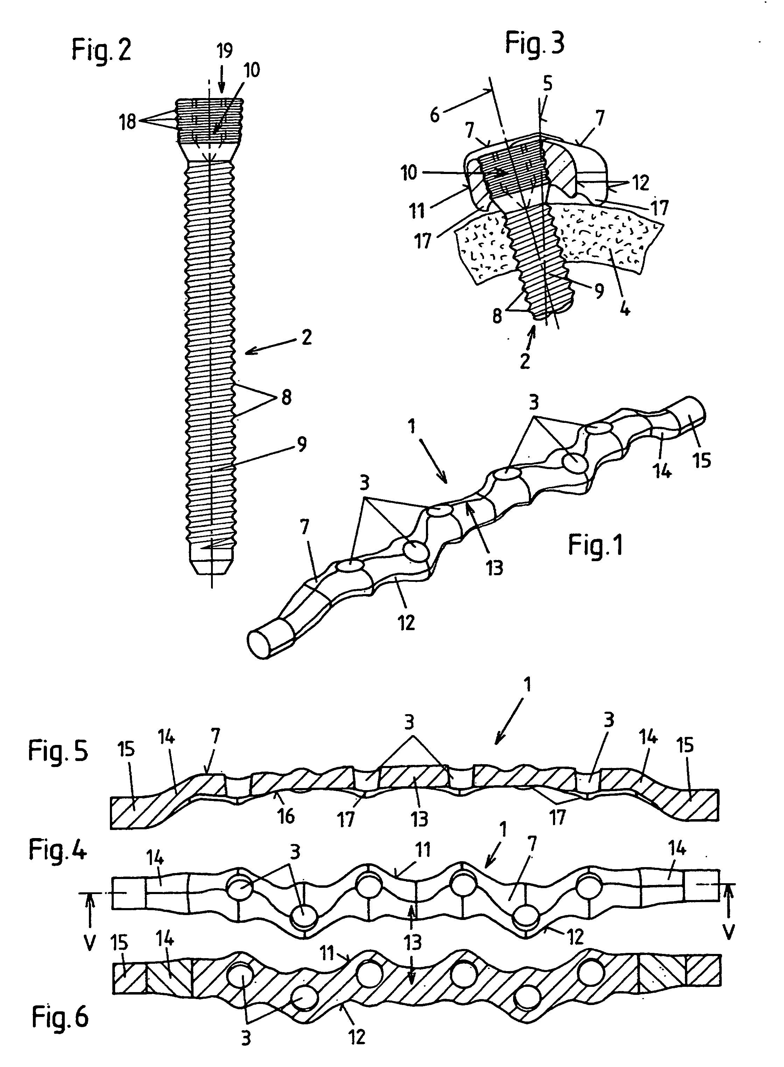

Um eine optimale Konvergenz der einzudrehenden Schrauben 2 zu erreichen (siehe auch die Darstellung in Fig. 3) sind die Mittelachsen 6 der Löcher 3 und somit im eingedrehten Zustand der Schrauben 2 auch deren Mittelachsen in einem spitzen Winkel W zu der gedachten Mittelebene 5 der Platte 1 ausgerichtet. Vorteilhaft schließen die Mittelachsen 6 der Löcher 3 mit der gedachten Mittelebene 5 der Platte 1 einen spitzen Winkel W von annähemd 15° ein.

Die Platte 1 und vorteilhafterweise auch die Schraube 2 sind aus faserverstärkten Thermoplasten hergestellt. Die Fertigung kann in einem Pressverfahren, beispielsweise in einem Fließpressverfahren oder in einem Gegentaktfließpressverfahren, erfolgen. Dabei ist die Anisotropie der elastischen Eigenschaften der Platte 1 auf die Elastizität oder Steifigkeit des Knochens 4 einstellbar. Bei der Herstellung der Platte ist die Anisotropie der elastischen Eigenschaften gemäß der Formel E-Modul(längs) : E-Modul(tangential) = 0,3 bis 0,7 einstellbar. Als optimal wird dabei ein Mittelwert von 0,5 angesehen.

Damit eine ordnungsgemäße Klemmwirkung des Kopfes 10 der Schraube 2 in dem entsprechenden Loch 3 der Platte 1 erzielt werden kann, um also die erforderliche Steifigkeit dieser Verbindung zu bewerkstelligen, wird vorteilhaft der Kopf 10 der Schraube 2 mit einem Gewinde 18 versehen. Auf dem an sich in korrespondierender Konstruktion mit dem Loch 3 konisch ausgeführten Kopf 10 der Schraube ist in optimaler Weise ein Feingewinde vorgesehen. Bei einer zweigängigen Ausgestaltung des Feingewindes wird eine dem Gewinde 8 auf dem Schaft 9 angepasste Gewindesteigung erreicht und außerdem eine feste Klemmwirkung des Kopfes 10 der Schraube 2 in bezug auf die Platte 1. Wenn außerdem der Kopf 10 der Schraube 2 im Bereich des Gewindes 18 mit einer oder mit mehreren Längsrille(n) versehen ist, ist eine Möglichkeit zur Aufnahme von Gewebedebris geschaffen. Beim Eindrehen der Schraube 2 wird in den letzten paar Umdrehungen plötzlich eine Erhöhung des Drehmomentes bewirkt, wobei dieses höhere Drehmoment durch den gegenseitigen Eingriff der Wandung des Loches 3 und des Kopfes 10 der Schraube 2 zustandekommt. Dies bedeutet eine optimale Klemmwirkung zwischen Schraube 2 und Platte 1, wobei es niemals zu einem Überdrehen des Gewindes 8 im Knochen 4 kommen kann.

Um eine weitere Möglichkeit der Verdrehsicherung zu bewerkstelligen, ist die Schraube 2 an ihrem mit einem Gewinde 8 versehenen Schaft 9 im Querschnitt unrund, z.B. trilobular, ausgeführt. Solche Querschnittsformen werden in der Regel als "Gleichdick" bezeichnet Im Rahmen der Erfindung wäre es denkbar, auch den Bereich des Kopfes 10 der Schraube 2 im Querschnitt unrund auszuführen. Der Kopf 10 der Schraube 2 ist mit einem Innenwerkzeugangriff 19 versehen, was für die Montage und auch für die Demontage solcher Schrauben vorteilhaft ist. Eine vorteilhafte Ausgestaltung sieht dabei einen Innenwerkzeugangriff 19 mit vier von einer mittigen Öffnung radial nach außen geführten bogenförmigen Ausbuchtungen vor. Auf diese Weise ist eine sehr günstige Übertragung des Drehmomentes möglich.

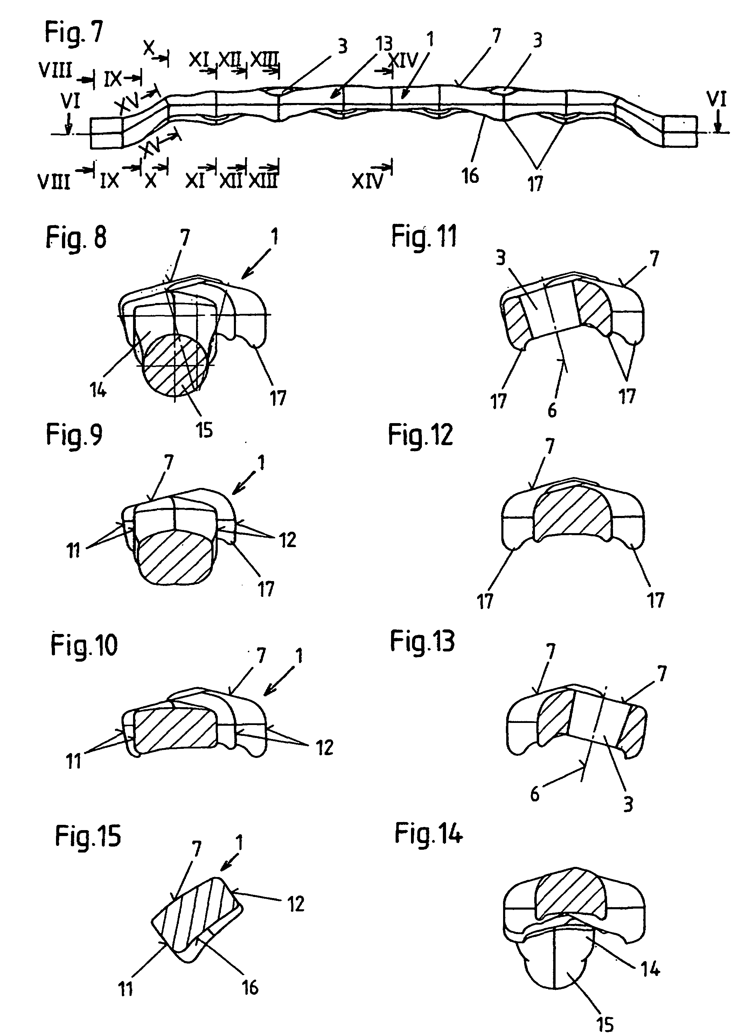

Die Schnittdarsteltungen in den Fig. 8 bis 15 bedürfen keiner weiteren Erläuterungen. Die technischen Details sind bereits im einzelnen aus der vorstehenden Beschreibung ersichtlich, wobei die Schnittdarstellung für sich selbstredend sind.

Sowohl an der Platte 1 als auch an der eingesetzten Schraube 2 sind wesentliche erfinderische Merkmale gegeben, die in ihrer Summe zusätzlich noch eine optimale Erhöhung der Tauglichkeit von Implantaten mit sich bringen.

## Patentansprüche

1. Implantat für Osteosynthesen, bestehend aus einer mit mehreren in Längsrichtung aufeinander folgenden Löchern (3) versehenen Platte (1) und durch die Löcher (3) in der Platte (1) hindurch einsetzbaren und in bestimmungsgemäßer Lage in einen Knochen eindrehbaren Schrauben (2), wobei zumindest ein Großteil der zur Aufnahme der Schrauben (2) bestimmten Löcher (3) bezogen auf eine gedachte Mittelebene (5) der Platte (1) alternierend nach außen versetzt sind, wobei die Mittelachsen (6) der Löcher (3) mit der gedachten Mittelebene (5) der Platte (1) einen spitzen Winkel (W) einschließen wobei die Löcher (3) von der bestimmungsgemäß außen liegenden Oberfläche (7) der Platte (1) ausgehend sich verjüngen, wobei die Löcher (3) der Platte (1) sich konisch verjüngen und wobei die Platte (1) in deren Längsrichtung gesehen mehrfach verwunden ausgeführt ist und im Bereich der einzelnen Löcher (3) die Hauptausrichtung der Platte (1) quer zur Längserstreckung der Platte (1) jeweils rechtwinklig zur Mittelachse (6) des entsprechenden Loches (3) verläuft, **dadurch gekennzeichnet, dass** die Schrauben (2) einen zu den von den Löchem (3) gebildeten Abschnitten korrespondierenden, sich konisch zu dem mit einem Gewinde (8) versehenen Schaft (9) hin verjüngenden Kopf (10) aufweisen, der kraftund/oder formschlüssig in den Löchern (3) fixierbar ist, und dass die Platte (1) von der Seite her gesehen leicht gebogen ausgeführt ist, wobei eine durch die Enden (14) der Platte (1) gelegte Sehne Abstand von einem Mittelabschnitt (13) der Platte (1) hat.

2. Implantat nach Anspruch 1, **dadurch gekennzeichnet, dass** die Seitenbegrenzungen (11,12) der Platte (1) den versetzten Löchern (3) und den Außenkonturen der Löcher (3) folgen, so dass die Platte (1) über deren Länge in Draufsicht gesehen einen im wesentlichen wellenförmigen Verlauf aufweist.

3. Implantat nach Anspruch 1 oder 2, **dadurch gekennzeichnet, dass** die dem Mittelabschnitt (13) der Platte (1) bezogen auf deren Länge nächstliegenden beiden Löcher (3) derselben Seitenbegrenzung (11,12) der Platte (1) zugewandt sind.

4. Implantat nach einem der Ansprüche 1 bis 3, **dadurch gekennzeichnet, dass** die gedachte Mittelebene (5) der Platte (1) auch die Mittelebene des Mittelabschnittes (13) der Platte (1) ist.

5. Implantat nach einem der Ansprüche 1 bis 4, **dadurch gekennzeichnet, dass** die Größe der Querschnittsfläche der Platte (1) zumindest annähernd über deren ganze Länge konstant ist

6. Implantat nach einem der Ansprüche 1 bis 4, **dadurch gekennzeichnet, dass** die Querschnittsfläche der Platte (1) im Bereich des Mittelabschnittes (13) größer ausgeführt ist als in den anderen Abschnitten der Platte (1).

7. Implantat nach einem der Ansprüche 1 bis 6, **dadurch gekennzeichnet, dass** der Abstand der Löcher (3) in Längsrichtung der Platte (1) gesehen von den Plattenenden ausgehend gleich ist, daß jedoch der Abstand zwischen den beiden an den Mittelabschnitt (13) anschließenden Löchern (3) größer ist.

8. Implantat nach einem der Ansprüche 1 bis 7, **dadurch gekennzeichnet, dass** an der Unterseite (16) der Platte (1) ein- oder beidseitig eines jeden Loches (3) nahe der Seitenbe grenzungen (11,12) auskragende Nocken (17) ausgebildet sind.

9. Implantat nach einem der Ansprüche 1 bis 8, **dadurch gekennzeichnet, dass** die beiden Enden (14) und die Kanten und Übergänge der Platte (1) flach und abgerundet ausgeführt sind.

10. Implantat nach einem der vorstehenden Ansprüche, **dadurch gekennzeichnet, dass** die Mittelachsen (6) der Löcher (3) mit der gedachten Mittelebene (5) der Platte (1) einen spitzen Winkel (W) von 15° einschließen.

11. Implantat nach einem der vorstehenden Ansprüche, **dadurch gekennzeichnet, dass** die Platte (1) aus faserverstärkten Thermoplasten hergestellt ist und die Anisotropie der elastischen Eigenschaften der Platte (1) auf die Elastizität oder Steifigkeit des Knochens (4) eingestellt ist.

12. Implantat nach Anspruch 11, **dadurch gekennzeichnet, dass** die Anisotropie der elastischen Eigenschaften der Platte (1) gemäß der Formel E-Modul(längs) : E-Modul(tangential) = 0,3 bis 0,7 eingestellt ist.

13. Implantat nach einem der vorstehenden Ansprüche, **dadurch gekennzeichnet, dass** der Kopf (10) der Schraube (2) mit einem Gewinde (18) versehen ist

14. Implantat nach Anspruch 13, **dadurch gekennzeichnet, dass** der Kopf (10) der Schraube (2) mit einem Feingewinde versehen ist

15. Implantat nach den Ansprüchen 13 und 14, **dadurch gekennzeichnet, dass** der Kopf (10) der Schraube (2) im Bereich des Gewindes (18) mit einer oder mit mehreren Längsrille(n) versehen ist.

16. Implantat nach einem der vorstehenden Ansprüche, **dadurch gekennzeichnet, dass** die Schraube (2) an ihrem mit einem Gewinde (8) versehenen Schaft (9) im Querschnitt unrund, z.B. trilobular, ausgeführt ist.

17. Implantat nach einem der vorstehenden Ansprüche, **dadurch gekennzeichnet, dass** der Kopf (10) der Schraube (2) mit einem Innenwerkzeugangriff (19) mit vier von einer mittigen Öffnung radial nach außen geführten bogenförmigen Ausbuchtungen versehen ist.

## Claims

1. Implant for osteosyntheses, consisting of a plate (1), which is provided with a plurality of holes (3), which follow one another in the longitudinal direction, and screws (2), which can be inserted through the holes (3) in the plate (1) and screwed into a bone in a specified position, wherein at least the majority of the holes (3) intended for accommodating the screws (2) are staggered outwards in an alternating manner relative to an imaginary centre plane (5) of the plate (1), wherein the centre axes (6) of the holes (3) form an acute angle (W) with the imaginary centre plane (5) of the plate (1), wherein the holes (3) taper from the surface (7) of the plate (1) specified as the outer surface, wherein the holes (3) of the plate (1) taper conically, and wherein the plate (1), viewed in its longitudinal direction, is repeatedly twisted, and the main orientation of the plate (1) in the region of each of the individual holes (3) extends transversely to the longitudinal extent of the plate (1) at a right angle to the centre axis (6) of the corresponding hole (3), **characterised in that** the screws (2) comprise a head (10) which corresponds to the portions formed by the holes (3), tapers conically towards the shank (9), which is provided with a thread (8), and can be fixed by friction type locking and/or positive locking in the holes (3), and that, viewed from the side, the plate (1) is slightly curved, wherein a chord laid through the ends (14) of the plate (1) is at a spacing from the centre portion (13) of the plate (1).

2. Implant according to Claim 1, **characterised in that** the lateral boundaries (11, 12) of the plate (1) follow the staggered holes (3) and the outer contours of the holes (3), so that, viewed over its length in plan view, the plate (1) has a substantially undulatory shape.

3. Implant according to Claim 1 or 2, **characterised in that** the two holes (3) which are nearest to the centre portion (13) of the plate (1), related to the length thereof, face the same lateral boundary (11, 12) of the plate (1).

4. Implant according to any one of Claims 1 to 3, **characterised in that** the imaginary centre plane (5) of the plate (1) is also the centre plane of the centre portion (13) of the plate (1).

5. Implant according to any one of Claims 1 to 4, **characterised in that** the size of the cross-sectional area of the plate (1) is constant at least approximately over the entire length thereof.

6. Implant according to any one of Claims 1 to 4, **characterised in that** the cross-sectional area of the plate (1) is larger in the region of the centre portion (13) than in the other portions of the plate (1).

7. Implant according to any one of Claims 1 to 6, **characterised in that**, viewed in the longitudinal direction of the plate (1), starting from the plate ends, the spacing of the holes (3) is uniform, that, however, the spacing between the two holes (3) adjacent to the centre portion (13) is greater.

8. Implant according to any one of Claims 1 to 7, **characterised in that** bosses (17), which protrude near the lateral boundaries (11, 12), are formed at the underside (16) of the plate (1) on one or both side(s) of each hole (3) .

9. Implant according to any one of Claims 1 to 8, **characterised in that** the two ends (14) and the edges and transitions of the plate (1) are flat and rounded.

10. Implant according to any one of the preceding Claims, **characterised in that** the centre axes (6) of the holes (3) form an acute angle (W) of 15E with the imaginary centre plane (5) of the plate (1).

11. Implant according to any one of the preceding Claims, **characterised in that** the plate (1) is made of fibre-reinforced thermoplastics, and the anisotropy of the elastic properties of the plate (1) is adapted to the elasticity or stiffness of the bone (4).

12. Implant according to Claim 11, **characterised in that** the anisotropy of the elastic properties of the plate (1) is adapted according to the formula modulus of elasticity (longitudinal) : modulus of elasticity (tangential) = 0.3 to 0.7.

13. Implant according to any one of the preceding Claims, **characterised in that** the head (10) of the screw (2) is provided with a thread (18).

14. Implant according to Claim 13, **characterised in that** the head (10) of the screw (2) is provided with a fine thread.

15. Implant according to Claims 13 and 14, **characterised in that** the head (10) of the screw (2) is provided with one or more longitudinal flute(s) in the region of the thread (18).

16. Implant according to any one of the preceding Claims, **characterised in that** the screw (2) has a non-circular, e.g. trilobular, cross section at its shank (9) provided with a thread (8).

17. Implant according to any one of the preceding Claims, **characterised in that** the head (10) of the screw (2) is provided with an internal tool application part (19) with four arcuate bulges extending radially outwards from a central opening.

## Revendications

1. Implant pour ostéosynthèse composé d'une plaque (11) munie de plusieurs trous (3) se suivant dans la direction longitudinale, et de vis qui se placent dans les trous (3) de la plaque (1) et se vissent dans une position déterminée dans un os,
au moins une grande partie des trous (3) destinés à recevoir les vis (2) étant décalée vers l'extérieur de manière alternée par rapport à un plan médian géométrique (3) de la plaque (1),
les axes (6) des trous (3) faisant un angle aigu (W) par rapport au plan médian géométrique (5) de la plaque (1),
les trous (3) allant suivant une forme conique en diminuant à partir de la surface supérieure (7) de la plaque (1), surface qui par définition est située à l'extérieur, alors que la plaque (1) vue dans sa direction longitudinale comporte plusieurs torsions et au niveau des différents trous (3), la direction principale de la plaque (1), transversalement à l'extension longitudinale de la plaque (1) est chaque fois perpendiculaire à l'axe médian (6) du trou correspondant (3),
**caractérisé en ce que**
les vis (2) ont une tête (10) correspondant au segment formé par les trous (3), en diminuant de manière conique vers une tige (9) munie d'un filetage (8), cette tige se fixant par une liaison de force et/ou de forme dans les trous (3) et
la plaque (1), vue de côté, est légèrement courbe,
une corde passant par les extrémités (14) de la plaque (1), ayant une certaine distance par rapport à un segment central (13) de la plaque (1).

2. Implant selon la revendication 1,
**caractérisé en ce que**
les limites latérales (11, 12) de la plaque (1) suivent les trous décalés (3) et les contours extérieurs des trous (3) de sorte qu'en vue de dessus, dans la direction longitudinale, la plaque (1) présente un tracé essentiellement ondulé.

3. Implant selon la revendication 1,
**caractérisé en ce que**
les deux trous (3) les plus proches dans le sens de la longueur du segment central (13) de la plaque (1), sont tournés vers la même limite latérale (11, 12) de la plaque (1).

4. Implant selon l'une des revendications 1 à 3,
**caractérisé en ce que**
le plan médian géométrique (5) de la plaque (1) est également le plan médian du segment central (13) de la plaque (1).

5. Implant selon l'une des revendications 1 à 4,
**caractérisé en ce que**
la dimension de la surface de la section de la plaque (1) est au moins sensiblement constante sur toute sa longueur.

6. Implant selon l'une des revendications 1 à 4,
**caractérisé en ce que**
la surface de la section de la plaque (1) au niveau du segment central (13) est plus grande que dans les autres segments de la plaque (1).

7. Implant selon l'une des revendications 1 à 6,
**caractérisé en ce que**
la distance des trous (3) vue dans la direction longitudinale de la plaque (1) est la même partant des extrémités de la plaque mais la distance entre les deux trous (3) adjacents au segment central (13) est plus grande.

8. Implant selon l'une des revendications 1 à 7,
**caractérisé en ce que**
le côté inférieur (16) de la plaque (1) comporte, d'un côté ou des deux côtés de chaque trou (3), des bossages (17) venant en saillie à proximité des limites latérales (11, 12).

9. Implant selon l'une des revendications 1 à 8,
**caractérisé en ce que**
les deux extrémités (14) et les arêtes et les transitions de la plaque (1) sont plates et arrondies.

10. Implant selon l'une des revendications précédentes,
**caractérisé en ce que**
les axes géométriques (6) des trous (3) font avec le plan médian géométrique (5) de la plaque (1), un angle aigu (W) égal à 15°.

11. Implant selon l'une des revendications précédentes,
**caractérisé en ce que**
la plaque (1) est fabriquée en matière thermoplastique renforcée par des fibres et l'anisotropie des propriétés élastiques de la plaque (1) est réglée en fonction de l'élasticité ou de la rigidité de l'os (4).

12. Implant selon la revendication 11,
**caractérisé en ce que**
l'anisotropie des propriétés élastiques de la plaque (1) est réglée selon la formule module d'élasticité longitudinal/module d'élasticité tangentiel = 0,3 à 0,7.

13. Implant selon l'une des revendications précédentes,
**caractérisé en ce que**
la tête (10) de la vis (2) est munie d'un filetage (18).

14. Implant selon la revendication 13,
**caractérisé en ce que**
la tête (10) de la vis (2) est munie d'un filetage fin.

15. Implant selon les revendications 13 et 14,
**caractérisé en ce que**
la tête (10) de la vis (2) est munie d'une ou plusieurs rainures longitudinales au niveau du filetage (18).

16. Implant selon l'une des revendications précédentes,
**caractérisé en ce que**
la section de la vis (2) au niveau de sa tige (9) munie d'un filetage (8) a une section non ronde, par exemple trilobée.

17. Implant selon l'une des revendications précédentes,
**caractérisé en ce que**
la tête (10) de la vis (2) est munie d'une prise pour outil intérieur (19) avec quatre cavités en arc de cercle, guidées radialement vers l'extérieur à partir d'un orifice central.
